# EUROPEAN PATENT SPECIFICATION

(11) **EP 0 756 850 B1**
(45) Date of publication and mention of the grant of the patent: **19.05.2004**
(21) Application number: 96304708.9
(22) Date of filing: 26.06.1996
(51) Int. Cl.: A61B 5/09, A63B 23/18

(54) **A training device and a ventilatory capacity meter**
Übungsvorrichtung und Atmungskapazitätmesser
Dispositif d'exercice et appareil de mesure de la capacité respiratoire

(30) Priority: 11.07.1995 GB 9514108
(43) Date of publication of application: 05.02.1997
(73) Proprietor: CLEMENT CLARKE INTERNATIONAL LIMITED, Harlow Essex, CM20 2ED (GB)
(72) Inventor: Sanders, Mark Jeremy, Church Langley, Harlow, Essex CM17 9PT (GB)
(74) Representative: Naylor, Matthew John

(56) References cited:
- WO-A-96/40376
- DE-A- 4 319 562
- US-A- 1 424 732
- US-A- 3 628 280
- US-A- 4 444 202

## Description

This invention relates to a combination of a training device and a ventilatory capacity meter, in particular a training device suitable for training a subject to use a ventilatory capacity meter to measure maximum parameters such as peak flow rate of exhalation.

A known type of meter for obtaining a measure of the peak flow rate of exhalation includes a tubular body having a inlet opening at one end through which a subject blows air into a chamber to displace a piston within the body, against the force of a spring. The chamber has an outlet opening for the air being blown into it, which takes the form of a slot running in the direction of the piston displacement and which is increasingly opened as the piston is displaced. A pointer runs in the slot and is displaced by the piston as it moves forward against the spring force. When the intensity of exhalation falls, the piston moves back and the pointer remains at the position of maximum displacement of the piston, so giving an indication of the maximum flow rate obtained in the exhalation. An example of such a meter is described in GB 1463814.

To obtain an accurate measurement of peak flow rate of exhalation, it is important that the subject exhales as rapidly as it is possible for them to do so. If they do not do this, the reading is not one of peak flow rate but simply an arbitrary flow rate that they are capable of achieving. This necessary rapid exhalation is not easy and requires some amount of practice to master.

Particularly in the case of children, where it has been observed that they have difficulty in perfecting the method of using peak flow meters, training devices are used, which give a visual or audible indication that the subject has exhaled at or above a predetermined rate. Such training devices provide an incentive for children to perform to their maximum and are recognised by doctors and specialist nurses as valuable tools for the training process.

For example, it is known to use an adjustable whistle which can be adjusted to require a certain predetermined flow rate through it before it sounds. In this way, a child can be encouraged to exhale as rapidly as they are able to in order to make the whistle sound. Another known training device is an electronic device having an LCD display which is used to display images which react to the subject's breath, encouraging them to blow as rapidly as they can.

Whilst these devices may be used to encourage children to exhale rapidly, they suffer from the drawback that they do not allow the child to be trained on the peak flow meter itself. Thus, when a child is trained on such a training device and is subsequently given a peak flow meter the differences between the two devices may be significant, resulting in a drop in the child's performance. A further disadvantage is the cost of these separate training devices, which may even be greater than that of the peak flow meters themselves.

It is also known to provide a specially adapted peak flow meter which includes an integral training device. In one example, a pop-out crocodile is provided. When the child exhales into the device at a predetermined flow rate the crocodile pops out from the body, against the force of a spring. The spring strength can be adjusted to alter the predetermined flow rate at which the crocodile pops out. Whilst this integral training device overcomes some of the problems associated with the known specific training devices, it increases the cost of the peak flow meter in which it is incorporated.

The present invention aims to overcome or ameliorate at least some of the above problems, by providing a combination of a training device and a ventilatory capacity meter, which can be used for training subjects on the meter itself, yet is inexpensive and requires little or no modification to existing meter designs.

Accordingly, the present invention provides combination of a training device and a ventilatory capacity meter as set out in claim 1.

The present invention is particularly suitable for use in training for peak flow exhalations.

If the known peak flow meter described above is provided with the training device, as the subject blows into the meter, the piston is driven along the chamber by a distance corresponding to the flow rate of exhalation. As the piston moves along the chamber the outlet slot in the side of the chamber is increasingly opened. After the piston has been driven a sufficient distance down the chamber to open a portion of the slot adjacent which the indicator element lies, the air escaping through the slot will operate the indicator element.

Preferably therefore, the mounting element is arranged such that it is possible to vary the position of the indicator element along the meter, ie. in the direction of piston movement, allowing its position to be selected in accordance with the anticipated peak flow rate of exhalation of the subject. In this way, the same training device can be set to the position most suitable for the peak flow rate of exhalation of any subject.

The mounting element is preferably a resilient clip element, for instance a band which is capable of frictionally gripping the peak flow meter for which it is intended. The band may be mountable on the meter by sliding it over the end of the body of the meter, however, more preferably, an open ring band is provided for additional resilient flexibility so that it can be easily snapped on and off the body of the meter, the body of the meter being received through the opening of the ring band.

In preferred embodiments of the training device of the present invention the band of the clip element is arranged so as to be slidable along the body of the peak flow meter, allowing the device to be easily positioned in accordance with the anticipated peak flow rate of the subject.

If required or desired, locating means may be provided for positively locating the band in any one of a number of discrete locations along the length of the meter. Means may also be provided for positively locating the indicator device at a required angular orientation about the longitudinal axis of the meter, relative to the slot outlet opening.

The indicator element may be a visible indicator, or an audible indicator, or both. Examples of suitable indicator elements include whistles, streamers, windmills, propellers, etc. However, the present invention is not limited to any particular indicator element. In fact, it is an advantage of the present invention that the indicator element may be selected to be any shape and/or colour, for example, to capture more readily a child's interest and thus provide a greater incentive.

A preferred embodiment of the training device of the present invention will now be described in detail, by way of example only, with reference to the accompanying drawings, in which:
Fig. 1 is a part-cut-away side elevational view of a peak flow meter carrying a training device, and
Fig. 2 is a front elevational view of the training device shown in Fig. 1.

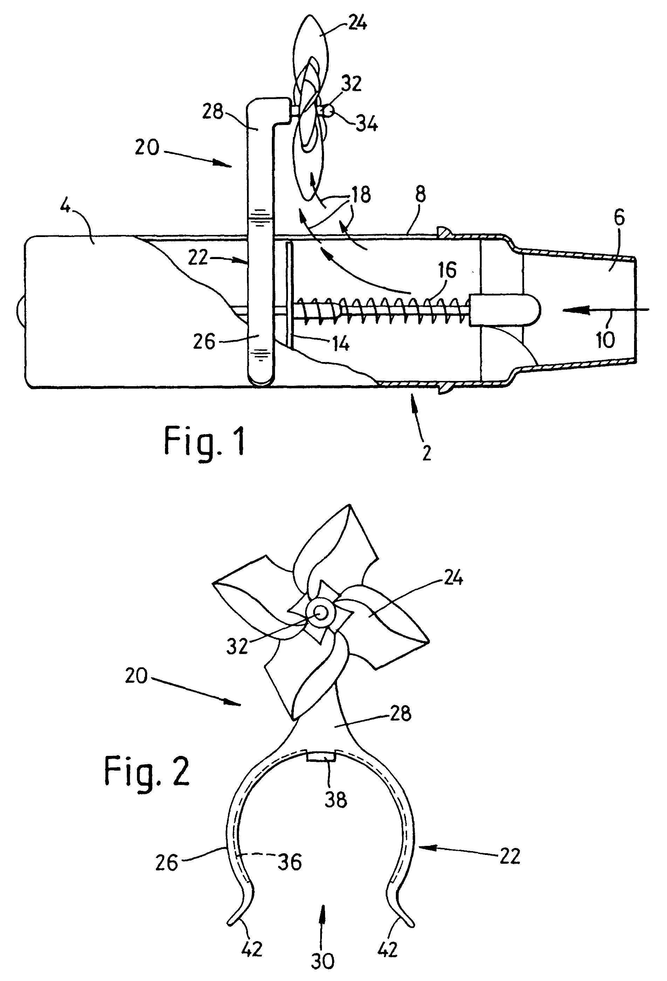

The peak flow meter 2 shown in Fig. 1 is of the known type already referred to. It includes a cylindrical body 40 having an inlet mouthpiece 6 at one end and a longitudinal slot 8, which serves as an outlet opening, in its side wall. When air (indicated by arrow 10) is blown into the meter through the mouthpiece 6, a piston 14 is forced along the interior of the body 4, away from the mouthpiece 6, against the force of a spring 16. The displacement of the piston 14 is dependent on the air flow rate. The air blown into the meter escapes through the slot 8, as indicated by the further arrows 18. A pointer (not shown) is held slidably in the slot and is moved by the piston to indicate its maximum displacement.

A training device 20 for the peak flow meter 3 includes an attaching clip element 22 and an indicator element 24 in the form of a windmill rotor. Rotors such as the one illustrated, formed from a stellate blank having the tips folded over to the centre to form the vanes, are well known in other contexts, such as children's toys and as decoration for drinking straws, and so will not be described in any detail here.

The clip element 22 includes a generally circular, open band 26 and a support stem 28 which protrudes radially outwardly from the band 26 at a point diametrically opposed to the opening 30 in the band. A spindle 32 is provided at the outer end of the support stem 28 and protrudes substantially perpendicularly to the support stem. The windmill rotor 24 is freely rotatably mounted on the spindle 32 after being snap-fitted over end protuberance 34.

The band is resiliently flexible and it grips the body of the meter frictionally. In addition, however, a circumferential slot 36 is provided on the radially inner surface of the band 26 for engaging circumferential ribs 36a provided on the surface of the meter body 4 spaced along it in the longitudinal direction, enabling the training device 20 to be positively located in any one of a number of discrete positions.

To help the correct location of the windmill rotor near to the slot 8, a lug 38 protrudes radially inwardly from the inner surface of the band 26 to engage in the slot 8. The dimensions of the lug 38 are selected such that when the band 26 is clipped on the meter body the lug protrudes into the slot 8 a sufficient distance to positively locate the windmill rotor 24 circumferentially of the body, but does not protrude so far as to interfere with the movement of the piston 14 or any pointer device carried in the slot.

Thus, when the training device 20 is mounted on the meter body it can be positively located in both the longitudinal and angular directions relative to the slot 8, with the windmill rotor near to the slot.

The band 26 and the support stem 28, including the spindle 32, are formed of a plastics material (e.g. polypropylene) as a unitary component. However, other suitably resilient or flexible materials may be used and the band and support stem may be formed as two or more separable components joined together by a snap-fit connector, for instance. In this way it is possible to provide a variety of shapes and sizes of clip elements and/or a variety of indicator elements which can be selected independently of one another and then combined to provide a training device appropriate to the specific subject and for fitting onto the peak flow meter in question.

In use, the training device 20 can be simply attached to and removed from the body of the meter with a snap action. The ends 42 of the band 26 have a reflex curvature, helping to guide the band easily over the body when it is clipped in place. While mounted on the body, the training device can be easily slid along the body to position it in the desired location, in accordance with the anticipated peak flow rate of exhalation of the subject.

When the subject blows into the mouthpiece 6, if the rate of exhalation moves the piston 14 to the point at which the portion of the slot 8 adjacent the windmill rotor 24 is opened. The air flow 18 from the slot then drives the rotor causing it to turn on the spindle 32. This provides a visual indication to the subject that they have achieved their goal.

The training device can be slid along the body 4 away from the inlet opening 56 so that the subject must then blow harder to displace the piston sufficiently to allow the windmill motor to be driven. On the other hand, if the subject is unable to move the rotor at a particular position of adjustment, it can be moved back towards the mouthpiece 6. In this way, a subject can be trained to deliver its best peak flow rate of exhalation into the meter.

Thus the present invention provides a detachably mountable training device for use in combination with a peak flow meter, providing the desired training incentive. A further advantage associated with the present invention, when the meter has a slot type outlet opening, is that the position of the training device along the meter can be adjusted for different subjects, according to their anticipated peak flow rate of exhalation. Furthermore, the device can be so arranged that it can be used with pre-existing meters.

## Claims

1. A combination of a training device (20) and a ventilatory capacity meter (2), the training device being releasably attachable to the ventilatory capacity meter, the ventilatory capacity meter being of the type including a chamber having an inlet opening through which air can be blown into the chamber, a piston (14) which is displaceable along the axis of elongation of the chamber in response to air blown into the chamber, an outlet (8) at a side of the chamber increasingly opened as the piston is displaced by blown air to allow the air to escape from the chamber, and a pointer held slidably in the outlet (8) which is moved by the piston to indicate the maximum displacement of the piston, wherein the training device comprises a mounting element (22) detachably mounted on the meter and an indicator element (24) supported by the mounting element and operable by a flow of air, the indicator element being supported near to the outlet (8) of the meter to be operated by the air escaping through the outlet.

2. A combination according to claim 1 wherein the mounting element (22) permits variation of the position at which the indicator element (24) is mounted on the meter.

3. A combination according to claim 1 or claim 2 wherein the mounting element is a resilient clip element (22) arranged to grip the meter.

4. A combination according to claim 3 wherein the clip element is in the form of an open ring band (26).

5. A combination according to any one of the preceding claims comprising means (38) for positively locating the device at a predetermined angular orientation with respect to the meter.

6. A combination according to any one of the preceding claims wherein the indicator element (24) is a visible or audible indicator.

7. A combination according to any one of claims 1 to 6 wherein means (36) are provided for locating the device at any of a plurality of predetermined positions on the meter body.

## Patentansprüche

1. Kombination aus einer Übungsvorrichtung (20) und einem Atmungskapazitätsmesser (2), wobei die Übungsvorrichtung abnehmbar am Atmungskapazitätsmesser befestigt werden kann und der Atmungskapazitätsmesser eine Kammer mit einer Eintassöffnung, durch die Luft in die Kammer eingeblasen werden kann, einen Kolben (14), der entlang der Längsachse der Kammer in Reaktion auf in die Kammer eingeblasene Luft verschoben werden kann, einen Auslass (8) an einer Seite der Kammer, der zunehmend geöffnet wird, wenn der Kolben durch die eingeblasene Luft verlagert wird, damit die Luft aus der Kammer austreten kann, sowie einen Zeiger beinhaltet, der gleitend im Auslass (8) gehalten und durch den Kolben bewegt wird, um die maximale Verlagerung des Kolbens anzuzeigen, worin die Übungsvorrichtung ein Befestigungselement (22), das abnehmbar auf dem Messer befestigt ist, und ein Anzeigeelement (24) umfasst, das vom Befestigungselement gehalten wird und durch einen Luftstrom betrieben werden kann, wobei das Anzeigeelement in der Nähe des Auslasses (8) des Messers angeordnet ist, um durch die aus dem Auslass austretende Luft betrieben zu werden.

2. Kombination nach Anspruch 1, worin das Befestigungselement (22) eine Veränderung der Position zulässt, in der das Anzeigeelement (24) auf dem Messer befestigt ist.

3. Kombination nach Anspruch 1 oder 2, worin das Befestigungelement ein elastisches Klemmelement (22) ist, das angeordnet ist, um an den Messer angeklemmt zu werden.

4. Kombination nach Anspruch 3, worin das Klemmelement in Form eines offenen Ringbands (26) ausgebildet ist.

5. Kombination nach einem der vorhergehenden Ansprüche, die Mittel (38) umfasst, um die Vorrichtung in einer vorbestimmten Winkelorientierung in Bezug auf den Messer formschlüssig anzuordnen.

6. Kombination nach einem der vorhergehenden Ansprüche, worin das Anzeigeelement (24) ein sichtbarer oder hörbarer Anzeiger ist.

7. Kombination nach einem der Ansprüche 1 bis 6, worin Mittel (36) bereitgestellt sind, um die Vorrichtung in einer beliebigen einer Vielzahl von vorbestimmten Positionen auf dem Körper des Messers anzuordnen.

## Revendications

1. Combinaison d'un dispositif d'exercice (20) et d'un appareil de mesure (2) de la capacité respiratoire, le dispositif d'exercice pouvant être fixé relâchablement à l'appareil de mesure de la capacité respiratoire, l'appareil de mesure de la capacité respiratoire étant du type comprenant une chambre avec une ouverture d'entrée à travers laquelle l'air peut être soufflé dans la chambre, un piston (14) qui est déplaçable le long de l'axe d'allongement de la chambre en réponse à l'air soufflé dans la chambre, une sortie (8) à un côté de la chambre s'ouvrant progressivement lorsque le piston est déplacé par l'air soufflé pour permettre l'échappement de l'air de la chambre, et un indicateur retenu d'une manière coulissante dans la sortie (8) qui est déplacé par le piston pour indiquer le déplacement maximal du piston, où le dispositif d'exercice comprend un élément de montage (22) monté relâchablement sur l'appareil de mesure et un élément indicateur (24) supporté par l'élément de montage et actionnable par un écoulement d'air, l'élément indicateur étant supporté près de la sortie (8) de l'appareil de mesure pour être actionné par l'air s'échappant à travers la sortie.

2. Combinaison selon la revendication 1, où l'élément de montage (22) permet la variation de la position à laquelle l'élément indicateur (24) est monté sur l'appareil de mesure.

3. Combinaison selon la revendication 1 ou la revendication 2, où l'élément de montage est un élément de clip élastique (22) agencé pour saisir l'appareil de mesure.

4. Combinaison selon la revendication 3, où l'élément de clip est réalisé sous la forme d'une bande annulaire ouverte (26).

5. Combinaison selon l'une des revendications précédentes, comprenant un moyen (38) pour localiser positivement le dispositif à une orientation angulaire prédéterminée par rapport à l'appareil de mesure.

6. Combinaison selon l'une des revendications précédentes, où l'élément indicateur (24) est un indicateur visible ou audible.

7. Combinaison selon l'une des revendications 1 à 6, où des moyens (36) sont prévus pour localiser le dispositif à l'une quelconque d'une pluralité de positions prédéterminées sur le corps de l'appareil de mesure.
